# EUROPÄISCHE PATENTSCHRIFT

(11) **EP 4 097 213 B1**
(45) Veröffentlichungstag und Bekanntmachung des Hinweises auf die Patenterteilung: **06.03.2024**
(21) Anmeldenummer: 21701460.4
(22) Anmeldetag: 20.01.2021
(51) Int. Cl.: C12M 1/107, C12M 1/06, C12M 1/34, C12M 1/36

(54) **FERMENTERBEHÄLTER EINER BIOGASANLAGE MIT WENIGSTENS EINEM HÖHENVERSTELLBAREN RÜHRWERK**
FERMENTER VESSEL OF A BIOGAS PLANT HAVING AT LEAST ONE HEIGHT-ADJUSTABLE AGITATOR
CUVE DE FERMENTATION D'UNE INSTALLATION DE BIOGAZ COMPORTANT AU MOINS UN AGITATEUR RÉGLABLE EN HAUTEUR

(30) Priorität: 30.01.2020 DE 102020102264
(43) Veröffentlichungstag der Anmeldung: 07.12.2022
(73) Patentinhaber: Niederbacher, Michael, 39031 Bruneck (IT)
(72) Erfinder: Niederbacher, Michael, 39031 Bruneck (IT)
(74) Vertreter: Liebl, Thomas
(86) Internationale Anmeldenummer: PCT/EP2021/051127
(87) Internationale Veröffentlichungsnummer: WO 2021/151746

(56) Entgegenhaltungen:
- EP-A1- 1 130 084
- EP-A1- 2 213 720
- EP-A1- 2 689 830
- DE-A1-102007 009 451
- DE-A1-102014 116 246
- DE-U1-202009 005 024

## Beschreibung

Die Erfindung betrifft einen Fermenterbehälter einer Biogasanlage mit wenigstens einem höhenverstellbaren Rührwerk nach dem Oberbegriff des Anspruches 1 sowie ein Verfahren zum Betreiben eines Fermenterbehälters nach dem Oberbegriff des Anspruchs 16.

Bei einem gattungsgemäßen bekannten Fermenterbehälter einer Biogasanlage (DE 10 2007 009451 A1) ist ein Rührwerk als Tauchmotorrührwerk an einer senkrechten Führungsschiene mit Führungselementen und mittels eines Zugseils an einer ortsfesten Seilrolle im oberen Führungsschienenbereich höhenverstellbar gehalten. Das Rührwerk ist mit einem steuerbaren Seilrollen-Motorantrieb als Höhenstellmotor durch Auf- oder Abwickeln des Seils höhenverstellbar.

Zudem ist hier eine Füllstandsmessvorrichtung vorgesehen, mit der eine Füllhöhe eines Substrats erfasst und ein entsprechendes Füllstands-Messsignal als Höhen-Istwertsignal erzeugt wird. Zudem ist eine Steuereinrichtung vorgesehen, der das Füllstands-Messsignal zugeführt wird, wobei ein Höhenstellmotor für das Tauchmotorrührgerät so angesteuert wird, dass dieses bei Bedarf soweit abgesenkt wird, dass die Rührflügel des Tauchmotorrührwerks vollständig im Substrat eingetaucht sind. Damit ist weder eine Abschaltung des Seilrollen-Motorantriebs bzw. des Höhenstellmotors an einer unteren oder oberen Endlage des Rührwerks noch eine Erkennung und Erfassung einer Schwimm- oder Sinkschicht und gegebenenfalls deren Dicke möglich.

Weiter ist aus der DE 20 2009 005024 U1 eine Höhenverstellvorrichtung mit einem Führungssystem und einem Höhenverstellmittel für ein Rührwerk in einem Gärbehälter bekannt, wobei das Höhenverstellmittel durch einen Hydraulikzylinder und ein außerhalb des Behälters angeordnetes Hydraulikdruck-Erzeugungsmittel gebildet ist.

Zudem ist aus der EP 2 213 720 A1 eine Biogasanlage mit einem in einem Fermenterbehälter an einem Standrohr höhenverstellbar geführten Tauch-Rührwerk bekannt, wobei zur Höhenverstellung des Tauch-Rührwerks eine Stelleinrichtung vorgesehen ist, die eine Spindel sowie an einem Schiebeteil eine Spindelmutter aufweist.

Aufgabe der Erfindung ist es, einen gattungsgemäßen Fermenterbehälter einer Biogasanlage mit wenigstens einem höhenverstellbaren Rührwerk so weiterzubilden, dass eine Höhenverstellung des Rührwerks mit relativ geringem Aufwand und relativ einfachen Maßnahmen auf funktionssichere Weise durchgeführt werden kann.

Diese Aufgabe wird mit den Merkmalen der unabhängigen Ansprüche gelöst.

Gemäß Anspruch 1 ist ein Fermenterbehälter einer Biogasanlage mit wenigstens einem höhenverstellbaren Rührwerk vorgesehen, wobei das Rührwerk im Fermenterbehälter mit einem Führungselement an einer in Hochachsenrichtung in etwa senkrecht ausgerichteten Führungsschiene geführt und mittels eines Zugseils höhenverstellbar gehalten ist. Erfindungsgemäß ist eine Zugkraft-Messeinrichtung zur direkten oder indirekten Messung der Zugkraft auf das Zugseil entsprechend einer Seilspannung vorgesehen, dergestalt, dass mit der Zugkraft-Messeinrichtung bei einer gesteuerten Höhenverstellung des Rührwerks variierende Zugkraft-Messsignale entsprechend variierender Zugkräfte erzeugt und einer Auswerteeinrichtung zugeführt werden.

Dadurch besteht die Möglichkeit, eine Abschaltung der Höhenverstellung an Endlagen des Rührwerks zu realisieren und/oder Höhenlagen und/oder Dicken und/oder Konsistenzen von Schwimm- und/oder Sinkschichten im Substrat zu ermitteln und gegebenenfalls mit diesen Informationen Maßnahmen in der Anlagensteuerung, insbesondere Höhenstellungen, Richtungsstellungen, Laufzeiten und Drehzahlen, etc. eines oder mehrerer Tauchmotorrührwerke geeignet an den Fermentationsprozess anzupassen.

Weiter ist vorgesehen, dass das Rührwerk mittels des Zugseils an einer Seilrolle höhenverstellbar gehalten ist, wobei das Rührwerk mit einem steuerbaren Seilrollen-Motorantrieb als Höhenstellmotor durch Auf- oder Abwickeln des Zugseils höhenverstellbar ist. Erfindungsgemäß ist in der Auswerteeinrichtung wenigstens ein Zugkraft-Schwellwert vorgegeben, bei dessen Erreichen der Seilrollen-Motorantrieb selbsttätig abschaltet. Wenn beispielsweise bei einem Absenkvorgang des Rührwerks ein unterer vorgegebener Anschlagpunkt im Fermenterbehälter erreicht wird oder das Rührwerk von oben her auf eine Schwimm- oder Sinkschicht mit besonders hoher Konsistenz stößt, so fällt die Zugkraft auf das Zugseil nach unten entsprechend der reduzierten Seilspannung stark ab und der Seilrollen-Motorantrieb wird von der Auswerteeinrichtung selbsttätig abgeschaltet.

Entsprechend kann die Abschaltfunktion gegebenenfalls mit einer Störungsmeldung auch bei einer Höhenverstellung des Rührwerks von unten nach oben erfolgen, wenn ein vorgegebener oberer Anschlag an der Führungsschiene oder eine weitgehend undurchdringliche Schwimm- oder Sinkschicht von unten her erreicht ist und die Seilspannung dadurch ansteigt.

Beim Durchlaufen einer Sink- bzw. Schwimmschicht, von oben nach unten oder von unten nach oben, welche noch ohne Störungsfunktion und Abschaltung mit dem Rührwerk durchfahren werden kann, ist gegebenenfalls durch die dortige Verringerung oder Erhöhung der Seilspannung beim Durchlaufen sowohl die Dicke als auch die Konsistenz einer Sink- oder Schwimmschicht ermittelbar.

Einfache Abschaltvorgänge in Verbindung mit hohen Seilspannungsänderungen können gegebenenfalls über einfache Endschalter und feste Verdrahtungen realisiert werden, ohne dass komplexe Auswertungen in vorzugsweise programmierbaren digitalen Steuerungen vorgenommen werden müssen.

Eine Auswerteeinrichtung zur Auswertung von gemessenen Seilspannungssignalen kann regelmäßig einfach in eine meist ohnehin vorhandene Anlagensteuerung für die Biogasanlage integriert werden.

Die Seilrolle ist gemäß einer besonders bevorzugten Ausgestaltung ortsfest am Fermenterbehälter angeordnet, vorzugsweise an einem oberen Führungsschienenbereich der Führungsschiene angeordnet. Die ortsfeste Anordnung insbesondere am oberen Führungsschienenbereich bewirkt eine gute Zugänglichkeit und günstige geometrische Verhältnisse im Hinblick auf die Seilführung zum Rührwerk.

In einer bevorzugten Ausführungsform der Erfindung ist die Zugkraft-Messeinrichtung Bestandteil des steuerbaren Seilrollen-Motorantriebs, wobei durch Messung einer vom Seilrollen-Motorantrieb während einer Höhenverstellung aufgenommen Stellenergie, vorzugsweise unter Berücksichtigung der Stellrichtung nach unten oder nach oben, jeweils ein aktuelles Zugkraft-Messsignal bzw. Seilspannungs-Messsignal erzeugt wird, welches dann in der Auswerteeinrichtung gegebenenfalls für weitere Maßnahmen ausgewertet wird.

Bevorzugt wird hier als steuerbaren Seilrollen-Motorantrieb ein elektrischer Motorantrieb verwendet, wobei ein Strommessgerät, vorzugsweise ein Amperemeter, vorgesehen ist, mit dem die Stromaufnahme während einer Höhenverstellung zur Erzeugung eines aktuellen Zugkraft-Messsignals gemessen und der Auswerteeinrichtung zugeführt wird.

Weiter kann alternativ oder redundant eine andere Zugkraft-Messeinrichtung verwendet werden, welche z.B. ortsfest im Bereich der Führungsschiene und/oder z.B. unterhalb einer Seilrolle angeordnet sein kann. Eine solche Zugkraft-Messeinrichtung weist bevorzugt eine Umlenkvorrichtung mit wenigstens einem Umlenkelement für das Zugseil aus seiner vertikalen Spannrichtung auf, wodurch eine vom Zugseil ausgehende Querkraft auf das wenigstens eine Umlenkelement entsprechend einer aktuellen Seilspannung erzeugt wird. Je höher die Zugkaft und damit die Seilspannung ist, umso höher ist auch die aus der Umlenkung resultierende Querkraft. Diese Querkraft wird mit einem Sensorelement gemessen und als Zugkraft-Messsignal einer Auswerteeinrichtung zugeführt.

In einer bevorzugten konkreten Ausführungsform weist die Umlenkvorrichtung als Umlenkelemente mehrere Umlenkrollen, vorzugsweise drei Umlenkrollen in einer Dreiecksanordnung, auf, wobei eine, bezogen auf die Hochachsenrichtung, obere Umlenkrolle und eine untere Umlenkrolle mit Anlageflächen in der vertikalen Seilspannungsrichtung liegen. Eine mittlere Umlenkrolle ist, vorzugsweise achsparallel, zwischen der oberen und der unteren Umlenkrolle in Querrichtung versetzt und beweglich geführt angeordnet. Das Zugseil ist gegenüberliegend zu den Anlageflächen der oberen Umlenkrolle und der unteren Umlenkrolle um die mittlere Umlenkrolle geführt, so dass diese bei einer Seilbelastung wegen ihres Querversatzes mit einer horizontalen Querkraft in Richtung der vertikalen Seilspannungsrichtung gedrängt wird. Vorzugsweise weist die Zugkraft-Messeinrichtung als Sensorelement einen Drucksensor auf, der mit seiner drucksensitiven Fläche mit der Halterung der mittleren Umlenkrolle zur Messung deren Querkraft und Erzeugung eines Zugkraft-Messsignals verbunden ist, wobei bevorzugt vorgesehen ist, dass der Drucksensor ortsfest an der Umlenkvorrichtung befestigt und/oder abgestützt ist.

Eine solche Zugkraftmesseinrichtung ist funktionssicher und relativ einfach aufgebaut sowie kostengünstig herstellbar.

Die Halterung und bewegliche Querführung der mittleren Umlenkrolle kann gemäß einer vorteilhaften Ausführungsvariante einfach mittels einer Schwinge ausgeführt werden, die schwenkbeweglich in Richtung des Drucksensors an der Umlenkvorrichtung, vorzugsweise an einem Lagerbock der oberen Umlenkrolle, befestigt ist. Für eine besonders stabile Anordnung ist die Schwinge als Gabelschwinge mit zwei voneinander beabstandeten Gabelteilen ausgebildet, zwischen denen die mittlere Umlenkrolle mit ihrer Achse angeordnet ist. Weiter kann vorteilhaft eine Abdeckung, beispielsweise als Sicherungsplatte, an der Außenseite der mittleren Umlenkrolle angebracht sein, die mit der Schwinge verbunden sein kann, um zu verhindern, dass das Zugseil, wenn es nicht unter Spannung steht von der mittleren Umlenkrolle springt.

Das Sensorelement als Drucksensor kann in der Art handelsüblicher Wiegezellen verwendet werden, die als piezoresistive Drucksensoren, insbesondere mit Dehnungsmessstreifen oder als piezoelektrische Drucksensoren oder als induktive Drucksensoren oder als kapazitive Drucksensoren auf dem Markt verfügbar sind.

In einer weiteren Ausgestaltung wird vorgeschlagen, dass an der Seilrolle und/oder am Seilrollenmotorantrieb ein Drehzahlsensor angeordnet ist, mit dem die aktuelle Anzahl der Seilwicklungen und damit die aktuell abgewickelte Seillänge in Verbindung mit der aktuellen Höhenposition des Rührwerks messbar ist. Ein damit erzeugtes Höhenpositionssignal kann in der Auswerteeinrichtung für eine Lagebestimmung und/oder Dickenbestimmung von Schwimm- und/oder Sinkschichten ausgewertet werden.

Gemäß einer besonders bevorzugten Ausführungsform können somit der Auswerteeinrichtung variierende Zugkraft-Messsignale zur Bestimmung der Höhenlage und/oder der Dicke und/oder der Konsistenz einer Schwimmschicht und/oder Sinkschicht im Substrat zuführbar sein, wobei bevorzugt vorgesehen ist, dass die dadurch erhaltene Information bezüglich der Höhenlage und/oder der Dicke und/oder der Konsistenz einer Schwimmschicht und/oder Sinkschicht im Substrat einer Anlagensteuerung für die Biogasanlage zugeführt wird und als Steuerungsparameter bei einer Steuerung der Biogasanlage, insbesondere bei einer Steuerung des Rührwerks, berücksichtigt und/oder auf einem Display angezeigt wird.

Weiter können als ein oder mehrere Rührwerke an sich bekannte Tauchmotorrührwerke eingesetzt werden mit einem vorzugsweise elektrischen oder hydraulischen oder pneumatischen Rührwerkmotor in einem Rohrgehäuse, aus dem an einer Seite axial eine Rührwerkwelle mit einem Propeller mit wenigstens einem Rührflügel ragt. Auch wenn dies die bevorzugte Ausführungsform ist, ist die Begrifflichkeit Rührwerk bei sämtlichen Ausführungen dieser Erfindung ausdrücklich in einem weiten Sinne zu verstehen und soll damit jede Art von Tauchgeräten umfasst sein, die zur Strömungserzeugung dienen, also ausdrücklich auch Tauchgeräte ohne Rührflügel, wie z.B. Tauchpumpen.

Gegenüberliegend zu den Rührwerkflügeln kann als Führungselement zudem ein die Führungsschiene umgreifender Führungsschlitten oder in diese eingreifender Führungsschlitten angeordnet sein, an dem das Rohrgehäuse befestigt und das Zugseil angeschlossen ist.

Die mit der erfindungsgemäßen Verfahrensführung erzielbaren Vorteile entsprechen denen des Fermenterbehälters. Insofern wird diesbezüglich zur Vermeidung von Wiederholungen auf die zuvor gemachten Ausführungen verwiesen.

Anhand einer Zeichnung wird die Erfindung lediglich beispielhaft näher erläutert.

Es zeigen:
- Figur 1: eine schematische Darstellung eines Fermenterbehälters einer Biogasanlage mit wenigstens einem höhenverstellbaren Rührwerk, und
- Figur 2: eine vergrößerte Darstellung einer Zugkraftmesseinrichtung an der Position A von Figur 1.

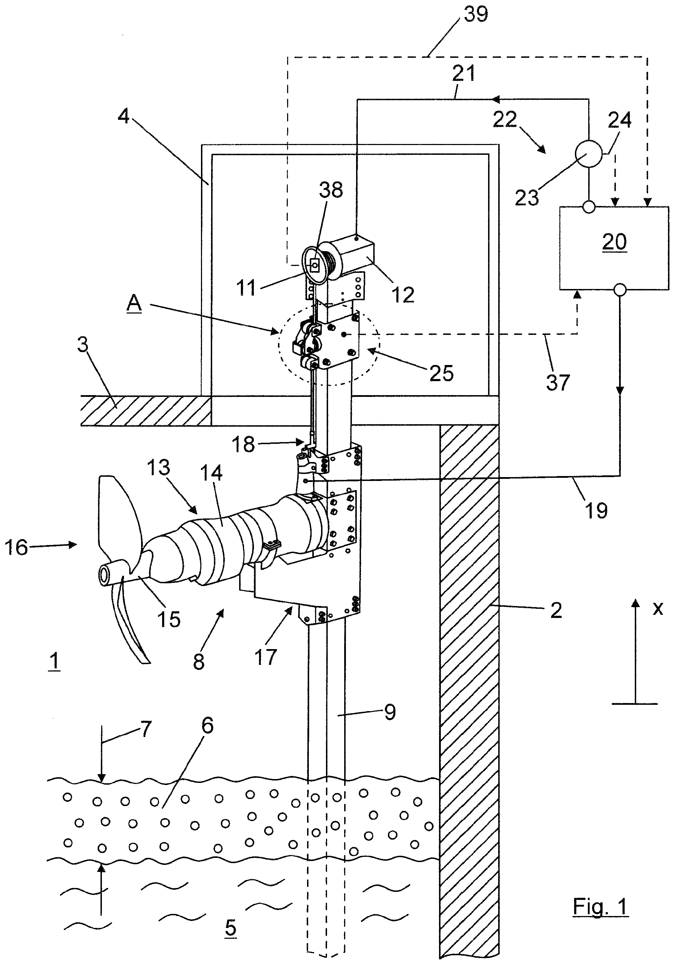

In Figur 1 ist stark schematisiert ein oberer Teilbereich eines Fermenterbehälters 1 einer Biogasanlage dargestellt mit einem Teilstück eines oberen Bereichs einer Außenwand 2 einer Folien- oder Deckenwand 3 und einem über einem Folien- oder Deckenausschnitt gasdicht angebrachten Serviceschacht 4 als Abdeckhaube. Der Fermenterbehälter 1 ist mit einem Substrat 5 aus flüssiger Biomasse bis in einen oberen Bereich gefüllt, wobei sich hier bereits eine Schwimmschicht 6 mit relativ dichter Konsistenz und einer Dicke 7 gebildet hat. Ein Rührwerk als Tauchmotorrührwerk 8 ist im Fermenterbehälter 1 in einem Seitenbereich an einer in Hochachsenrichtung x gesehen in etwa senkrecht ausgerichteten Führungsschiene 9 mittels eines Zugseils 10 an einer ortsfesten Seilrolle 11 im oberen Führungsschienenbereich gehalten. Das Tauchmotorrührwerk 8 ist mit einem steuerbaren, elektrischen Seilrollen-Motorantrieb 12 durch Auf- oder Abwickeln des Zugseils 10 höhenverstellbar an der Führungsschiene 9 gehalten.

Die Führungsschiene 9 ist einerseits im Folien- oder Deckenbereich bzw. im Bereich des Serviceschachtes 4 sowie andererseits am hier nicht dargestellten Boden des Fermenterbehälters 1 um ihre Achse schwenkbar gelagert, wobei diese Lagerstellen auf herkömmliche Weise ausgebildet sind unddeswegen nicht im Detail dargestellt sind.

Das Tauchmotorrührwerk 8 ist mit einem elektrischen Rührwerkmotor 13 in einem Rohrgehäuse 14 ausgerüstet, aus dem an einer Seite axial eine Rührwerkwelle 15 mit einem hier lediglich beispielhaft zweiflügligen Propeller 16 ragt. Gegenüberliegend zum Propeller 16 ist ein die Führungsschiene 9 umgreifender Führungsschlitten 17 als Führungselement angeordnet, an dem sowohl das Rohrgehäuse 14 als auch das untere Ende des Zugseils 10 an einem Anschlusspunkt 18 angeschlossen ist. Die elektrische Energieeinspeisung für den elektrischen Rührwerkmotor 13 erfolgt gesteuert/geregelt mit einer schematisch angedeuteten Stromleitung 19 von einem Steuergerät 20.

Auch der Seilrollen-Motorantrieb 12 ist hier ein elektrischer Motorantrieb und wird über das Steuergerät 20 und eine Stromleitung 21 mit Strom als Stellenergie versorgt.

Zur Realisierung einer Zugkraft-Messeinrichtung 22 zur indirekten Messung der Zugkraft auf das Zugseil 10 entsprechend einer Seilspannung wird während einer Höhenverstellung mittels eines (schematisch dargestellten) Amperemeters bzw. Strommessgerätes 23 die Stromaufnahme des Seilrollen-Motorantriebs 12 gemessen und über eine Signalleitung 24 dem Steuergerät 20 zugeführt. Das Steuergerät 20 enthält eine Auswerteeinrichtung, in der ein oder mehrere Zugkraft-Schwellwerte vorgebbar sind, bei deren Erreichen die Stromzufuhr zum Seilrollen-Motorantrieb 12 selbsttätig abschaltet. Zudem können in der Auswerteeinrichtung variierende Zugkraft-Messsignale zur Bestimmung der Höhenlage und/oder der Dicke 7 und/oder der Konsistenz von einer Schwimmschicht 6 oder einer Sinkschicht im Substrat 5 analysiert und ausgewertet werden, die dann insbesondere bei der Rührwerksteuerung berücksichtigt werden. Diese Informationen können gegebenenfalls auch auf einem (nicht dargestellten) Display angezeigt werden.

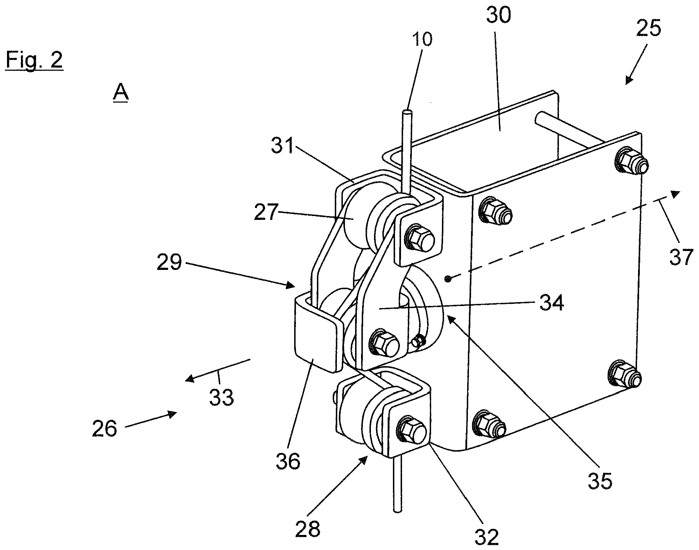

In Figur 1 ist zudem eine weitere alternative oder gegebenenfalls zusätzliche und damit redundante Zugkraft-Messeinrichtung 25 in dem mit strichpunktierter Linie umrandeten Bereich A eingezeichnet. Die alternative oder zusätzliche Zugkraft-Messeinrichtung 25 wird anhand der vergrößerten Darstellung in Figur 2 näher erläutert:
Die Zugkraft-Messeinrichtung 25 ist hier beispielhaft ortsfest im oberen Bereich der Führungsschiene 9 unterhalb der Seilrolle 11 angeordnet und weist eine Umlenkvorrichtung 26 mit im hier gezeigten Beispielfall wenigstens drei Umlenkrollen auf, nämlich einer oberen Umlenkrolle 27, einer unteren Umlenkrolle 28 und einer mittleren Umlenkrolle 29. Die Umlenkrollen 27, 28 und 29 sind im hier gezeigten Beispielfall somit in einer Dreiecksanordnungangeordnet. Die Zugkraft-Messeinrichtung 25 ist hier beispielhaft mit einem Spanngehäuse 30 an der Führungsschiene 9 unverschiebbar befestigt. An der Vorderwand des Spanngehäuses 30 kann, wie hier beispielhaft gezeigt, ein Lagerbock 31 für die obere Umlenkrolle 27 und ein Lagerbock 32 für die untere Umlenkrolle 28 angeordnet sein, wobei das Zugseil 10 senkrecht an der Rückseite auf die obere Umlenkrolle 27 von oben her geführt ist und von der unteren Umlenkrolle 28 wieder senkrecht nach unten weiterverläuft.

Die mittlere Umlenkrolle 29 kann, wie hier lediglich beispielhaft dargestellt, die gleichen Abmessungen wie die obere Umlenkrolle 27 und/oder die untere Umlenkrolle 28 aufweisen.

Im hier gezeigten konkreten Beispielfall ist die mittlere Umlenkrolle 29 achsparallel in Querrichtung (schematisch durch Pfeil 33 angedeutet) nach vorne versetzt angeordnet. Dabei ist die mittlere Umlenkrolle 29 an einer hier lediglich beispielhaft als Gabelschwinge 34 ausgebildeten Schwinge gelagert, die schwenkbeweglich am Lagerbock 31 angebunden bzw. mit diesem verbunden ist, so dass mittels der Gabelschwinge 34 die mittlere Umlenkrolle 29 in Querrichtung beweglich gehalten ist.

Das Zugseil 10 ist ersichtlich von der Rückseite der oberen Umlenkrolle 27 nach vorne über die mittlere Umlenkrolle 29 und dort wieder zurück über die hintere Seite der unteren Umlenkrolle 28 geführt, wodurch sich wegen des Querversatzes der mittleren Umlenkrolle 29 eine Querkraft in Richtung auf das Spanngehäuse 30 bei einer Zugbelastung des Zugseils 10 ergibt. Diese Querkraft wird mittels eines (handelsüblichen und nicht näher dargestellten) Drucksensors 35 als Maß für die Spannung des Zugseils 10 gemessen, wobei der Drucksensor 35 einerseits am Spanngehäuse 30 abgestützt und befestigt ist und andererseits die Halterung der mittleren Umlenkrolle 29 entsprechend der Gabelschwinge 34 mit einem Gabelschwingenteil auf der drucksensitiven Fläche des Drucksensors 35 aufliegt oder dort verbunden ist. Ersichtlich ist die Anordnung so ausgeführt, dass eine Querkraft möglichst senkrecht auf die drucksensitive Fläche des Drucksensors 35 wirkt.

An der Gabelschwinge 34 ist zudem ein Sicherungselement 36 als abgekröpftes Blechteil ausgebildet, das einen äußeren Bereich der mittleren Umlenkrolle 29 überdeckt, so dass ein entlastetes Zugseil 10 nicht von der mittleren Umlenkrolle 29 springen kann.

Ein mit dem Drucksensor 35 erzeugtes Drucksignal entsprechend einem Zugkraft-Messsignal auf das Zugseil 10 wird mit einer Signalleitung 37 dem Steuergerät 20 zugeführt und dort ausgewertet, wodurch gegebenenfalls Steuermaßnahmen eingeleitet oder angepasst werden, wie dies vorstehend in Verbindung mit der ersten Ausführung einer Zugkraft-Messeinrichtung 22 bereits beschrieben ist.

In einer weiteren Ausgestaltung ist am Seilrollen-Motorantrieb 12 oder hier an der Seilrolle 11 ein Drehzahlsensor 38 angeordnet, mit dem die aktuelle Anzahl der Seilwickelungen auf der Seilrolle 11 und damit die aktuell abgewickelte Seillänge in Verbindung mit der aktuellen Höhenposition des Tauchmotorrührwerks 8 gemessen wird. Ein entsprechendes Drehzahl- bzw. Höhenpositions-Signal wird mit der Signalleitung 39 dem Steuergerät 20 zugeführt, das Steuermaßnahmen auch unter Berücksichtigung der Höhenposition des Tauchmotorrührwerks 8 vornehmen kann.

### Bezugszeichenliste

- 1: Fermenterbehälter
- 2: Außenwand
- 3: Folien- oder Deckenwand
- 4: Serviceschacht
- 5: Substrat
- 6: Schwimmschicht
- 7: Dicke
- 8: Tauchmotorrührwerk
- 9: Führungsschiene
- 10: Zugseil
- 11: Seilrolle
- 12: Seilrollen-Motorantrieb
- 13: elektrischer Rührwerkmotor
- 14: Rohrgehäuse
- 15: Rührwerkwelle
- 16: Propeller
- 17: Führungsschlitten
- 18: Anschlusspunkt
- 19: Stromleitung
- 20: Steuergerät
- 21: Stromleitung
- 22: Zugkraft-Messeinrichtung
- 23: Strommessgerät
- 24: Signalleitung
- 25: Zugkraft-Messeinrichtung
- 26: Umlenkvorrichtung
- 27: obere Umlenkrolle
- 28: untere Umlenkrolle
- 29: mittlere Umlenkrolle
- 30: Spanngehäuse
- 31: Lagerbock
- 32: Lagerbock
- 33: Pfeil
- 34: Gabelschwinge
- 35: Drucksensor
- 36: Sicherungselement
- 37: Signalleitung
- 38: Drehzahlsensor
- 39: Signalleitung

## Patentansprüche

1. Fermenterbehälter (1) einer Biogasanlage,
mit wenigstens einem höhenverstellbaren Rührwerk (8), wobei das Rührwerk (8) im Fermenterbehälter (1) mit einem Führungselement (17) an einer in Hochachsenrichtung in etwa senkrecht ausgerichteten Führungsschiene (9) geführt und mittels eines Zugseils (10) höhenverstellbar gehalten ist,
wobei das Rührwerk (8) mittels des Zugseils (10) an einer Seilrolle (11) höhenverstellbar gehalten ist,
wobei das Rührwerk (8) mit einem steuerbaren Seilrollen-Motorantrieb (12) als Höhenstellmotor durch Auf- oder Abwickeln des Zugseils (10) höhenverstellbar ist,
**dadurch gekennzeichnet,**
**dass** eine Zugkraft-Messeinrichtung (22; 25) zur direkten oder indirekten Messung der Zugkraft auf das Zugseil (10) entsprechend einer Seilspannung vorgesehen ist, dergestalt, dass mit der Zugkraft-Messeinrichtung (22; 25) bei einer gesteuerten Höhenverstellung des Rührwerks (8) variierende Zugkraft-Messsignale entsprechend variierender Zugkräfte erzeugt und einer Auswerteeinrichtung (20) zugeführt werden, und
**dass** in der Auswerteeinrichtung (20) wenigstens ein Zugkraft-Schwellwert vorgegeben ist, bei dessen Erreichen der Seilrollen-Motorantrieb (12) selbsttätig abschaltet.

2. Fermenterbehälter nach Anspruch 1, **dadurch gekennzeichnet, dass** die Seilrolle (11) ortsfest am Fermenterbehälter (1) angeordnet ist, vorzugsweise an einem oberen Führungsschienenbereich der Führungsschiene (9) angeordnet ist.

3. Fermenterbehälter nach Anspruch 1 oder Anspruch 2, **dadurch gekennzeichnet,**
**dass** die Zugkraft-Messeinrichtung (22) Bestandteil des steuerbaren Seilrollen-Motorantriebs (12) ist, und
**dass** eine Stellenergie-Messeinheit vorgesehen ist, mit der eine vom Seilrollen-Motorantrieb (12) während einer Höhenverstellung aufgenommenen Stellenergie gemessen und daraus, vorzugsweise unter Berücksichtigung der Stellrichtung nach unten oder nach oben, jeweils ein aktuelles Zugkraft-Messsignal abgeleitet wird.

4. Fermenterbehälter nach einem der vorhergehenden Ansprüche, **dadurch gekennzeichnet,**
**dass** der steuerbare Seilrollen-Motorantrieb (12) ein elektrischer Motorantrieb ist, und
**dass** ein Strommessgerät (23), vorzugsweise ein Amperemeter, vorgesehen ist, mit dem die Stromaufnahme während einer Höhenverstellung zur Erzeugung eines aktuellen Zugkraft-Messsignals gemessen und der Auswerteeinrichtung (20) zugeführt wird.

5. Fermenterbehälter nach einem der vorhergehenden Ansprüche, **dadurch gekennzeichnet, dass** die Zugkraft-Messeinrichtung (25), vorzugsweise ortsfest, im Bereich der Führungsschiene (9) angeordnet ist, wobei bevorzugt vorgesehen ist, dass die Zugkraft-Messeinrichtung (25), bezogen auf die Hochachsenrichtung, unterhalb der Seilrolle (11) angeordnet ist.

6. Fermenterbehälter nach einem der vorhergehenden Ansprüche, **dadurch gekennzeichnet,**
**dass** die Zugkraft-Messeinrichtung (25) eine Umlenkvorrichtung (26) mit wenigstens einem Umlenkelement (29) für das Zugseil (10) aus seiner vertikalen Spannrichtung aufweist, und
**dass** ein Sensorelement (35) vorgesehen ist, mit dem die daraus resultierende Querkraft auf das wenigstens eine Umlenkelement (29) gemessen und als Zugkraft-Messsignal für die Seilspannung der Auswerteeinrichtung (20) zugeführt wird.

7. Fermenterbehälter nach Anspruch 6, **dadurch gekennzeichnet,**
**dass** die Umlenkvorrichtung (26) als Umlenkelemente mehrere Umlenkrollen, vorzugsweise wenigstens drei Umlenkrollen in einer Dreiecksanordnung, aufweist, wobei eine, bezogen auf die Hochachsenrichtung, obere Umlenkrolle (27) und eine untere Umlenkrolle (28) mit Anlageflächen in der vertikalen Seilspannungsrichtung liegen, und
**dass** eine mittlere Umlenkrolle (29), vorzugsweise achsparallel, zwischen der oberen Umlenkrolle (27) und der unteren Umlenkrolle (28) in Querrichtung versetzt und beweglich geführt angeordnet ist und das Zugseil (10) gegenüberliegend zu den Anlageflächen der oberen Umlenkrolle (27) und unteren Umlenkrolle (28) um die mittlere Umlenkrolle (29) geführt ist, so dass diese bei einer Seilbelastung mit einer horizontalen Querkraft in Richtung der vertikalen Seilspannungsrichtung gedrängt wird.

8. Fermenterbehälter nach Anspruch 7, **dadurch gekennzeichnet, dass** die Zugkraft-Messeinrichtung (25) als Sensorelement einen Drucksensor (35) aufweist, der mit seiner drucksensitiven Fläche mit der Halterung (34) der mittleren Umlenkrolle (29) zur Messung deren Querkraft und Erzeugung eines Zugkraft-Messsignals verbunden ist, wobei bevorzugt vorgesehen ist, dass der Drucksensor (35) ortsfest an der Umlenkvorrichtung (26) befestigt und/oder abgestützt ist.

9. Fermenterbehälter nach Anspruch 7 oder 8, **dadurch gekennzeichnet, dass** die Halterung und bewegliche Querführung der mittleren Umlenkrolle (29) mittels einer Schwinge (34) ausgeführt ist, die schwenkbeweglich in Richtung des Drucksensors (35) an der Umlenkvorrichtung (26), vorzugsweise an einem Lagerbock (31) der oberen Umlenkrolle (27), befestigt ist.

10. Fermenterbehälter nach Anspruch 9, **dadurch gekennzeichnet, dass** die Schwinge (34) als Gabelschwinge mit zwei voneinander beabstandeten Gabelteilen ausgebildet ist, zwischen denen die mittlere Umlenkrolle (29) mit ihrer Achse angeordnet ist.

11. Fermenterbehälter nach einem der Ansprüche 6 bis 10, **dadurch gekennzeichnet, dass** das Sensorelement als Drucksensor (35) in der Art einer Wiegezelle als piezoresistiver Drucksensor, insbesondere mit Dehnungsmessstreifen (DMS) oder als piezoelektrischer Drucksensor oder als induktiver Drucksensor oder als kapazitiver Drucksensor ausgebildet ist.

12. Fermenterbehälter nach einem der vorhergehenden Ansprüche, **dadurch gekennzeichnet, dass** an der Seilrolle (11) und/oder am Seilrollen-Motorantrieb (12) ein Drehzahlsensor (38) angeordnet ist, mit dem die aktuelle Anzahl der Seilwicklungen auf der Seilrolle (11) und damit die aktuell abgewickelte Seillänge in Verbindung mit der aktuellen Höhenposition des Rührwerks (8) messbar ist, so dass ein damit erzeugtes Höhenpositions-Signal der Auswerteeinrichtung (20) für eine Lagebestimmung und/oder Dickenbestimmung von Schwimm- und/oder Sinkschichten zugeführt wird.

13. Fermenterbehälter nach einem der vorhergehenden Ansprüche, **dadurch gekennzeichnet, dass** der Auswerteeinrichtung (20) variierende Zugkraft-Messsignale zur Bestimmung der Höhenlage und/oder der Dicke (7) und/oder der Konsistenz einer Schwimmschicht (6) und/oder Sinkschicht im Substrat (5) zuführbar sind, wobei bevorzugt vorgesehen ist, dass die dadurch erhaltene Information bezüglich der Höhenlage und/oder der Dicke (7) und/oder der Konsistenz einer Schwimmschicht (6) und/oder Sinkschicht im Substrat (5) einer Anlagensteuerung für die Biogasanlage zugeführt wird und als Steuerungsparameter bei einer Steuerung der Biogasanlage, insbesondere bei einer Steuerung des Rührwerks (8), berücksichtigt und/oder auf einem Display angezeigt wird.

14. Fermenterbehälter nach einem der vorhergehenden Ansprüche, **dadurch gekennzeichnet, dass** die Auswerteeinrichtung (20) in eine Anlagensteuerung für die Biogasanlage integriert ist.

15. Fermenterbehälter nach einem der vorhergehenden Ansprüche, **dadurch gekennzeichnet,**
**dass** das Rührwerk ein Tauchmotorrührwerk (8) mit einem vorzugsweise elektrischen oder hydraulischen oder pneumatischen Rührwerkmotor (13) in einem Rohrgehäuse (14) ist, aus dem an einer Seite axial eine Rührwerkwelle (15) mit einem Propeller (16) mit wenigstens einem Rührflügel ragt,
und/oder
**dass** gegenüberliegend zum Propeller (16) als Führungselement ein die Führungsschiene umgreifender oder in diese eingreifender Führungsschlitten (17) angeordnet ist, an dem das Rohrgehäuse (14) befestigt und das untere Ende des Zugseils (10) angeschlossen ist.

16. Verfahren zum Betreiben eines Fermenterbehälters (1) einer Biogasanlage,
mit wenigstens einem höhenverstellbaren Rührwerk (8), wobei das Rührwerk (8) im Fermenterbehälter (1) mit einem Führungselement (17) an einer in Hochachsenrichtung in etwa senkrecht ausgerichteten Führungsschiene (9) geführt und mittels eines Zugseils (10) höhenverstellbar gehalten ist,
wobei das Rührwerk (8) mittels des Zugseils (10) an einer Seilrolle (11) höhenverstellbar gehalten ist,
wobei das Rührwerk (8) mit einem steuerbaren Seilrollen-Motorantrieb (12) als Höhenstellmotor durch Auf- oder Abwickeln des Zugseils (10) höhenverstellbar ist,
**dadurch gekennzeichnet,**
**dass** eine Zugkraft-Messeinrichtung (22; 25) zur direkten oder indirekten Messung der Zugkraft auf das Zugseil (10) entsprechend einer Seilspannung vorgesehen ist, dergestalt, dass mit der Zugkraft-Messeinrichtung (22; 25) bei einer gesteuerten Höhenverstellung des Rührwerks (8) variierende Zugkraft-Messsignale entsprechend variierender Zugkräfte erzeugt und einer Auswerteeinrichtung (20) zugeführt werden, und
**dass** in der Auswerteeinrichtung (20) wenigstens ein Zugkraft-Schwellwert vorgegeben ist, bei dessen Erreichen der Seilrollen-Motorantrieb (12) selbsttätig abschaltet.

## Claims

1. A fermenter vessel (1) of a biogas plant,
having at least one height-adjustable agitator (8), wherein the agitator (8), in the fermenter vessel (1), is guided with a guide element (17) on a guide rail (9) oriented approximately vertically in a vertical axis direction and is held in height-adjustable manner by means of a traction cable (10),
with the agitator (8) being held in height-adjustable manner on a cable pulley (11) by means of the traction cable (10),
with the agitator (8) being height-adjustable by winding or unwinding the traction cable (10) with a controllable cable pulley motor drive (12) as a height actuator,
**characterized in that**
a traction force measuring device (22; 25) is provided for directly and/or indirectly measuring the traction force on the traction cable (10) in accordance with a cable tension, such that, by means of the traction force measuring device (22; 25), in the case of a controlled height adjustment of the agitator (8), varying traction force measurement signals are generated in accordance with varying traction forces and are supplied to an evaluation device (20) and at least one traction force threshold value is predefined in the evaluation device (20), wherein the cable pulley motor drive (12) shuts down automatically when said threshold value is reached.

2. The fermenter vessel according to claim 1, **characterized in that** the cable pulley (11) is arranged in a stationary manner on the fermenter vessel (1), preferably arranged on an upper guide rail region of the guide rail (9).

3. The fermenter vessel according to claim 1 or claim 2, **characterized in that** the traction force measuring device (22) is part of the controllable cable pulley motor drive (12), and
an actuator energy measuring unit is provided, by means of which an actuator energy consumed by the cable pulley motor drive (12) during a height adjustment is measured and a current traction force measurement signal is respectively derived therefrom, preferably taking into account the actuating direction downward or upward.

4. The fermenter vessel according to any one of the preceding claims, **characterized in that**
the controllable cable pulley motor drive (12) is an electric motor drive, and
a current measuring device (23), preferably an ammeter, is provided by means of which current consumption is measured during a height adjustment for generating a current traction force measurement signal and is supplied to the evaluation device (20).

5. The fermenter vessel according to any one of the preceding claims, **characterized in that** the traction force measuring device (25) is arranged preferably in a stationary manner in the region of the guide rail (9), wherein it is preferably provided for the traction force measuring device (25) to be arranged below the cable pulley (11) in relation to the vertical axis direction.

6. The fermenter vessel according to any one of the preceding claims, **characterized in that**
the traction force measuring device (25) has a deflection device (26) with at least one deflection element (29) for the traction cable (10) from its vertical clamping direction, and
a sensor element (35) is provided, by means of which the resulting transverse force on the at least one deflection element (29) is measured and supplied to the evaluation device (20) as a traction force measurement signal for the cable tension.

7. The fermenter vessel according to claim 6, **characterized in that** the deflection device (26) comprises a plurality of deflection rollers as deflection elements, preferably at least three deflection rollers in a triangular arrangement, wherein an - in relation to the vertical axis direction - upper deflection roller (27) and a lower deflection roller (28) are located with contact surfaces in the vertical cable tension direction, and
a central deflection roller (29) is arranged, preferably in an axially parallel manner, between the upper deflection roller (27) and the lower deflection roller (28) in a manner offset in the transverse direction and movably guided, and the traction cable (10) is guided opposite the contact surfaces of the upper deflection roller (27) and the lower deflection roller (28) about the central deflection roller (29), so that, when a load is exerted on the cable, the central deflection roller is pushed in the direction of the vertical cable tension direction with a horizontal transverse force.

8. The fermenter vessel according to claim 7, **characterized in that** the traction force measuring device (25) has a pressure sensor (35) as sensor element, which pressure sensor is connected with its pressure-sensitive surface to the mounting (34) of the central deflection roller (29) for measuring its transverse force and generating a traction force measurement signal, wherein it is preferably provided for the pressure sensor (35) to be fastened and/or supported in a stationary manner on the deflection device (26).

9. The fermenter vessel according to claim 7 or 8, **characterized in that** the mounting and movable transverse guidance of the central deflection roller (29) is carried out by means of a rocker arm (34) which is fastened pivotably in the direction of the pressure sensor (35) to the deflection device (26), preferably to a bearing block (31) of the upper deflection roller (27).

10. The fermenter vessel according to claim 9, **characterized in that** the rocker arm (34) is designed as a fork arm with two fork parts spaced apart from one another, between which the central deflection roller (29) is arranged with its axis.

11. The fermenter vessel according to any one of claims 6 to 10, **characterized in that** the sensor element is designed as a pressure sensor (35) in the manner of a weighing cell as a piezoresistive pressure sensor, in particular with strain gauges or as a piezoelectric pressure sensor or as an inductive pressure sensor or as a capacitive pressure sensor.

12. The fermenter vessel according to any one of the preceding claims, **characterized in that** a rotational speed sensor (38) is arranged on the cable pulley (11) and/or on the cable pulley motor drive (12), by means of which the current number of cable windings on the cable pulley (11) and thus the currently unwound cable length are measurable in conjunction with the current height position of the agitator (8), so that a height position signal generated therewith is supplied to the evaluation device (20) for determining the position and/or thickness of floating and/or sinking layers.

13. The fermenter vessel according to any one of the preceding claims, **characterized in that** varying traction force measurement signals are supplyable to the evaluation device (20) for determining the height position and/or the thickness (7) and/or the consistency of a floating layer (6) and/or sinking layer in the substrate (5), wherein it is preferably provided that the information obtained in this way with respect to the height position and/or the thickness (7) and/or the consistency of a floating layer (6) and/or sinking layer in the substrate (5) is supplied to a plant control for the biogas plant and is taken into account and/or shown on a display as a control parameter when controlling the biogas plant, in particular when controlling the agitator (8).

14. The fermenter vessel according to any one of the preceding claims, **characterized in that** the evaluation device (20) is integrated into a plant control for the biogas plant.

15. The fermenter vessel according to any one of the preceding claims, **characterized in that**
the agitator is a submersible motor agitator (8) having a preferably electric or hydraulic or pneumatic agitator motor (13) in a tubular housing (14), from which an agitator shaft (15) with a propeller (16) with at least one agitator blade projects axially on one side,
and/or
a guide carriage (17) encompassing the guide rail or engaging therein is arranged as a guide element opposite the propeller (16), to which guide carriage the tubular housing (14) is fastened and the lower end of the traction cable (10) is connected.

16. A method for operating a fermenter vessel (1) of a biogas plant,
having at least one height-adjustable agitator (8), wherein the agitator (8), in the fermenter vessel (1), is guided with a guide element (17) on a guide rail (9) oriented approximately vertically in a vertical axis direction and is held in height-adjustable manner by means of a traction cable (10),
with the agitator (8) being held in height-adjustable manner on a cable pulley (11) by means of the traction cable (10),
with the agitator (8) being height-adjustable by winding or unwinding the traction cable (10) with a controllable cable pulley motor drive (12) as a height actuator,
**characterized in that**
a traction force measuring device (22; 25) is provided for directly and/or indirectly measuring the traction force on the traction cable (10) in accordance with a cable tension, such that, by means of the traction force measuring device (22; 25), in the case of a controlled height adjustment of the agitator (8), varying traction force measurement signals are generated in accordance with varying traction forces and are supplied to an evaluation device (20), and at least one traction force threshold value is predefined in the evaluation device (20), wherein the cable pulley motor drive (12) shuts down automatically when said threshold value is reached.

## Revendications

1. Cuve de fermentation (1) d'une installation de biogaz,
Avec au moins un agitateur (8) réglable en hauteur, l'agitateur (8) étant guidé dans la cuve de fermentation (1) par un élément de guidage (17) sur un rail de guidage (9) orienté approximativement verticalement dans la direction de l'axe vertical et étant maintenu réglable en hauteur au moyen d'un câble de traction (10),
l'agitateur (8) étant maintenu sur une poulie à câble (11) de manière réglable en hauteur au moyen du câble de traction (10),
l'agitateur (8) étant réglable en hauteur par enroulement ou déroulement du câble de traction (10) au moyen d'un entraînement motorisé commandable (12) de poulie et faisant office de moteur de réglage en hauteur,
**caractérisée**
**en ce qu'**il est prévu un dispositif (22 ; 25) de mesure de la force de traction pour la mesure directe ou indirecte de la force de traction sur le câble de traction (10) en fonction d'une tension de câble, de telle sorte qu'avec le dispositif (22 ; 25) de mesure de la force de traction, lors d'un réglage en hauteur commandé de l'agitateur (8), des signaux de mesure de la force de traction variant en fonction de forces de traction variables sont générés et transmis à un dispositif d'évaluation (20), et
**en ce qu'**au moins une valeur seuil de force de traction est prédéfinie dans le dispositif d'évaluation (20), l'entraînement motorisé (12) de poulie s'arrêtant automatiquement lorsque cette valeur est atteinte.

2. Cuve de fermentation selon la revendication 1, **caractérisée en ce que** la poulie à câble (11) est agencée de manière fixe sur la cuve de fermentation (1), de préférence sur une zone de rail de guidage supérieure du rail de guidage (9).

3. Cuve de fermentation selon la revendication 1 ou 2, **caractérisée**
**en ce que** le dispositif (22) de mesure de la force de traction fait partie intégrante de l'entraînement motorisé commandable (12) de poulie, et
**en ce qu'**il est prévu une unité de mesure de l'énergie de réglage, avec laquelle une énergie de réglage absorbée par l'entraînement motorisé (12) de poulie pendant un réglage en hauteur est mesurée et un signal de mesure de la force de traction actuel en est dérivé, de préférence en tenant compte du sens de réglage vers le bas ou vers le haut.

4. Cuve de fermentation selon l'une des revendications précédentes, **caractérisée**
**en ce que** l'entraînement motorisé commandable (12) de poulie est un entraînement par moteur électrique, et
**en ce qu'**il est prévu un appareil de mesure de courant (23), de préférence un ampèremètre, avec lequel la consommation de courant est mesurée pendant un réglage en hauteur pour générer un signal de mesure de force de traction actuel et est transmise au dispositif d'évaluation (20).

5. Cuve de fermentation selon l'une des revendications précédentes, **caractérisée en ce que** le dispositif (25) de mesure de la force de traction est agencé, de préférence de manière fixe, dans la zone du rail de guidage (9), et il est de préférence prévu que le dispositif (25) de mesure de la force de traction est agencé, par rapport à la direction de l'axe vertical, au-dessous de la poulie à câble (11).

6. Cuve de fermentation selon l'une des revendications précédentes, **caractérisée**
**en ce que** le dispositif (25) de mesure de la force de traction présente un dispositif de renvoi (26) avec au moins un élément de renvoi (29) pour le câble de traction (10) à partir de sa direction de tension verticale, et
**en ce qu'**il est prévu un élément capteur (35) au moyen duquel la force transversale résultante sur ledit au moins un élément de renvoi (29) est mesurée et est transmise au dispositif d'évaluation (20) en tant que signal de mesure de la force de traction pour la tension du câble.

7. Cuve de fermentation selon la revendication 6, **caractérisée**
**en ce que** le dispositif de renvoi (26) présente, en tant qu'éléments de renvoi, plusieurs poulies de renvoi, de préférence au moins trois poulies de renvoi disposées en triangle, une poulie de renvoi supérieure (27) et une poulie de renvoi inférieure (28) étant situées, par rapport à la direction de l'axe vertical, avec des surfaces d'appui dans la direction verticale de tension du câble, et
**en ce qu'**une poulie médiane de renvoi (29), de préférence parallèle à l'axe, est agencée entre la poulie de renvoi supérieure (27) et la poulie de renvoi inférieure (28), en étant décalée dans le sens transversal et guidée de manière mobile, et le câble de traction (10) est guidé autour de la poulie médiane de renvoi (29), à l'opposé des surfaces d'appui de la poulie de renvoi supérieure (27) et de la poulie de renvoi inférieure (28), de sorte que celle-ci soit poussée vers la direction verticale de tension du câble par une force transversale horizontale, lors d'une charge du câble.

8. Cuve de fermentation selon la revendication 7, **caractérisée en ce que** le dispositif (25) de mesure de la force de traction présente, en tant qu'élément de détection, un capteur de pression (35) dont la surface sensible à la pression est reliée au support (34) de la poulie de renvoi centrale (29) pour mesurer sa force transversale et générer un signal de mesure de la force de traction, et il est de préférence prévu que le capteur de pression (35) soit fixé et/ou supporté de manière fixe sur le dispositif de renvoi (26).

9. Cuve de fermentation selon la revendication 7 ou 8, **caractérisée en ce que** le support et le guidage transversal mobile de la poulie médiane de renvoi (29) sont réalisés au moyen d'un bras oscillant (34) qui est fixé au dispositif de renvoi (26), de préférence à un support de palier (31) de la poulie de renvoi supérieure (27), de manière à pouvoir pivoter en direction du capteur de pression (35).

10. Cuve de fermentation selon la revendication 9, **caractérisée en ce que** le bras oscillant (34) est conçu comme un bras oscillant à fourche avec deux parties de fourche espacées l'une de l'autre, entre lesquelles la poulie de renvoi médiane (29) est agencée avec son axe.

11. Cuve de fermentation selon l'une des revendications 6 à 10, **caractérisée en ce que** l'élément de détection est sous la forme d'un capteur de pression (35) du type cellule de pesée en tant que capteur de pression piézo-résistif, en particulier avec une jauge de contrainte (DMS) ou en tant que capteur de pression piézoélectrique ou en tant que capteur de pression inductif ou en tant que capteur de pression capacitif.

12. Cuve de fermentation selon l'une des revendications précédentes, **caractérisée en ce qu'**un capteur de vitesse de rotation (38) est agencé sur la poulie à câble (11) et/ou sur l'entraînement motorisé (12) de poulie, au moyen duquel le nombre actuel de spires de câble sur la poulie à câble (11), et donc la longueur de câble actuellement déroulée, soient aptes à être mesurés en relation avec la position en hauteur actuelle de l'agitateur (8), de sorte qu'un signal de position en hauteur ainsi généré est transmis au dispositif d'évaluation (20) pour une détermination de la position et/ou de l'épaisseur de couches flottantes et/ou de couches immergées.

13. Cuve de fermentation selon l'une des revendications précédentes, **caractérisée en ce que** des signaux de mesure de force de traction variables sont aptes à être transmis au dispositif d'évaluation (20) pour déterminer la position en hauteur et/ou l'épaisseur (7) et/ou la consistance d'une couche flottante (6) et/ou d'une couche immergée dans le substrat (5), et il est de préférence prévu que l'information ainsi obtenue concernant la hauteur et/ou l'épaisseur (7) et/ou la consistance d'une couche flottante (6) et/ou d'une couche immergée dans le substrat (5) soit transmise à une commande d'installation pour l'installation de biogaz et qu'elle soit prise en compte comme paramètre de commande lors d'une commande de l'installation de biogaz, en particulier lors d'une commande de l'agitateur (8), et/ou qu'elle soit affichée sur un écran.

14. Cuve de fermentation selon l'une des revendications précédentes, **caractérisée en ce que** le dispositif d'évaluation (20) est intégré dans une commande d'installation pour l'installation de biogaz.

15. Cuve de fermentation selon l'une des revendications précédentes, **caractérisée**
**en ce que** l'agitateur est un agitateur à moteur immergé (8), avec un moteur d'agitateur (13), de préférence électrique ou hydraulique ou pneumatique, placé dans un boîtier tubulaire (14) duquel dépasse axialement d'un côté un arbre d'agitateur (15) avec une hélice (16) ayant au moins une pale d'agitateur,
et/ou
**en ce qu'**en face de l'hélice (16) est agencé en tant qu'élément de guidage un chariot de guidage (17) qui entoure le rail de guidage ou qui vient en engagement dans celui-ci, sur lequel est fixé le boîtier tubulaire (14) et auquel est raccordée l'extrémité inférieure du câble de traction (10).

16. Procédé pour faire fonctionner une cuve de fermentation (1) d'une installation de biogaz,
avec au moins un agitateur (8) réglable en hauteur, l'agitateur (8) étant guidé dans la cuve de fermentation (1) par un élément de guidage (17) sur un rail de guidage (9) orienté approximativement verticalement dans la direction de l'axe vertical et étant maintenu réglable en hauteur au moyen d'un câble de traction (10),
l'agitateur (8) étant maintenu sur une poulie à câble (11) de manière réglable en hauteur au moyen du câble de traction (10),
l'agitateur (8) étant réglable en hauteur par enroulement ou déroulement du câble de traction (10) au moyen d'un entraînement motorisé commandable (12) de poulie et faisant office de moteur de réglage en hauteur,
**caractérisé**
**en ce qu'**il est prévu un dispositif (22 ; 25) de mesure de la force de traction pour la mesure directe ou indirecte de la force de traction sur le câble de traction (10) en fonction d'une tension de câble, de telle sorte qu'avec le dispositif (22 ; 25) de mesure de la force de traction, lors d'un réglage en hauteur commandé de l'agitateur (8), des signaux de mesure de la force de traction variant en fonction de forces de traction variables sont générés et transmis à un dispositif d'évaluation (20), et
**en ce que** dans le dispositif d'évaluation (20) est prédéfinie au moins une valeur seuil de force de traction, l'entraînement motorisé (12) de poulie s'arrêtant automatiquement lorsque cette valeur est atteinte.
